# FASCICULE DE BREVET EUROPEEN

(11) **EP 1 549 741 B1**
(45) Date de publication et mention de la délivrance du brevet: **14.01.2009**
(21) Numéro de dépôt: 03807892.9
(22) Date de dépôt: 10.10.2003
(51) Int. Cl.: C12N 5/08, G01N 33/50, C07K 16/28, C07K 16/42, A61K 39/395, A61P 35/00, A61P 21/00, A61P 27/00, A61P 19/00, A61P 37/00, A61P 17/00, A61P 9/00

(54) **NOUVELLES CELLULES ENDOTHELIALES, ANTICORPS DIRIGES CONTRE CES CELLULES ET LEUR UTILISATION**
NEUARTIGE ENDOTHELZELLEN, GEGEN DIE ZELLEN GERICHTETE ANTIKÖRPER UND VERWENDUNG DAVON
NOVEL ENDOTHELIAL CELLS, ANTIBODIES AGAINST SAID CELLS AND USE THEREOF

(30) Priorité: 10.10.2002 FR 0212606
(43) Date de publication de la demande: 06.07.2005
(73) Titulaire: CENTRE NATIONAL DE LA RECHERCHE SCIENTIFIQUE (CNRS), 75794 Paris Cedex 16 (FR)
(72) Inventeur: PLOUËT, Jean, Pierre, F-75014 Paris (FR); PEDRON, Sandrine, Florence, F-31520 Ramonville Saint Agne (FR); MAITRE-BOUBE, Martine, Michèle, 31170 Tournefeuille (FR)
(74) Mandataire: Grosset-Fournier, Chantal Catherine
(86) Numéro de dépôt international: PCT/FR2003/002996
(87) Numéro de publication internationale: WO 2004/033674

(56) Documents cités:
- FR-A- 2 796 073
- US-A- 5 976 782
- HARTLAPP I ET AL: "Fibrocytes induce an angiogenic phenotype in cultured endothelial cells and promote angiogenesis in vivo." THE FASEB JOURNAL: OFFICIAL PUBLICATION OF THE FEDERATION OF AMERICAN SOCIETIES FOR EXPERIMENTAL BIOLOGY. UNITED STATES OCT 2001, vol. 15, no. 12, octobre 2001 (2001-10), pages 2215-2224, XP002249066 ISSN: 1530-6860
- HUTCHINGS HELEN ET AL: "Pigment epithelium-derived factor exerts opposite effects on endothelial cells of different phenotypes." BIOCHEMICAL AND BIOPHYSICAL RESEARCH COMMUNICATIONS, vol. 294, no. 4, 21 juin 2002 (2002-06-21), pages 764-769, XP002249067 June 21, 2002 ISSN: 0006-291X cité dans la demande
- MEADOWS K N ET AL: "Vascular endothelial growth factor induction of the angiogenic phenotype requires Ras activation." THE JOURNAL OF BIOLOGICAL CHEMISTRY. UNITED STATES 28 DEC 2001, vol. 276, no. 52, 28 décembre 2001 (2001-12-28), pages 49289-49298, XP002249068 ISSN: 0021-9258

## Description

La présente invention concerne de nouvelles cellules endothéliales, ainsi que des anticorps dirigés contre ces cellules.

Les cellules endothéliales tapissent l'intérieur de tous les vaisseaux sanguins de l'organisme. Ces cellules ont un phénotype quiescent, elles ne prolifèrent quasiment pas (seulement 0,01% des cellules endothéliales sont engagées dans la division cellulaire à un moment donné). Le rôle physiologique des cellules endothéliales est très vaste, elles participent à de nombreux phénomènes vitaux pour l'organisme : coagulation sanguine, maintien de la pression artérielle, recrutement de cellules circulantes, synthèse de facteurs de croissance. Quand un territoire requiert un apport accru en oxygène et en nutriments il réagit en relarguant des facteurs solubles qui convertissent le phénotype des cellules endothéliales en un phénotype activé, et des facteurs mitogènes pour les cellules endothéliales de phénotype activé. Ces cellules endothéliales acquièrent enfin un phénotype angiogénique et deviennent capables d'effectuer le programme d'angiogenèse, c'est-à-dire le développement de nouveaux vaisseaux sanguins à partir du réseau préexistant. Ce phénomène est normal et contrôlé lors d'une blessure ou dans la maturation du corps jaune de l'ovaire ; en revanche, il devient dangereux dans des situations pathologiques où l'angiogenèse n'est plus contrôlée, par exemple dans la rétinopathie diabétique, la polyarthrite et le développement tumoral.

Il est nécessaire qu'un agent thérapeutique s'attaque seulement aux cellules endothéliales participant à l'angiogenèse, c'est à dire aux cellules de phénotype angiogénique (Brooks et al., 1994), d'où l'importance de l'étude du phénotype angiogénique pour le développement de nouveaux outils afin de bloquer l'angiogenèse. Peu d'études ont jusqu'à maintenant été effectuées sur des cellules de phénotype angiogénique, une difficulté majeure étant l'impossibilité d'extraire des cellules de ce phénotype et de les maintenir différenciées en culture *in vitro.* Les cellules endothéliales sont classiquement cultivées en présence de FGF2, et donc comme toute cellule cultivée *in vitro,* elles perdent leur phénotype quiescent et acquièrent un phénotype activé lorsqu'elles prolifèrent dans une boîte de culture. En revanche le maintien du phénotype angiogénique pourrait être assuré par l'adjonction de FGF2 au milieu de culture (Boudreau et al., 1997 ; Battegay et al., 1994). Cependant, le rôle de FGF2 en pathologie est controversé.

A ce jour, on ne dispose pas de modèles d'étude de cellules endothéliales qui, bien que proliférant, soit miment le phénotype non activé (phénotype non angiogénique), soit miment le phénotype angiogénique.

La présente invention a pour but de fournir des cellules endothéliales dépendant exclusivement du VEGF.

La présente invention a pour but de fournir un procédé de préparation desdites cellules cultivées sur un tapis de gélatine ou de collagène et incubées ou non dans un milieu de culture dont la teneur en hormones et en facteurs de croissance est contrôlée.

L'expression "cellules endothéliales de phénotype non angiogénique" désigne des cellules incapables de s'organiser en tubes en culture dans un gel de collagène en présence de VEGF.

L'expression "cellules endothéliales de phénotype angiogénique" désigne des cellules capables de s'organiser en tubes en culture dans un gel de collagène en présence de VEGF.

L'expression "substances n'affectant substantiellement pas les cellules endothéliales de phénotype non angiogénique" désigne toute substance naturelle ou synthétique qui, ajoutée au milieu de culture, ne modifie pas l'activité des cellules de phénotype non angiogénique lors de l'un au moins des 3 tests suivants (décrits dans les exemples) : prolifération, migration, angiogenèse *in vitro.*

L'expression "substances n'affectant substantiellement pas les cellules endothéliales de phénotype angiogénique" désigne toute substance naturelle ou synthétique qui, ajoutée au milieu de culture, ne modifie pas l'activité des cellules de phénotype angiogénique lors de l'un au moins des 3 tests suivants (décrits dans les exemples) : prolifération, migration, angiogenèse *in vitro.*

La présente invention concerne
- des cellules endothéliales de phénotype angiogénique,
   présentant les propriétés suivantes :
- elles forment des tubes lorsqu'elles sont mises en présence du facteur de croissance VEGF, dans un gel de collagène
- elles prolifèrent sous l'action du VEGF,
- elles sont protégées de l'apoptose par le VEGF,
- leur expression de VEGFR-2 est 4 fois supérieure à celle des cellules correspondantes de phénotype non angiogénique,
   et caractérisées en ce qu'elles sont susceptibles de générer des anticorps présentant une activité antitumorale.

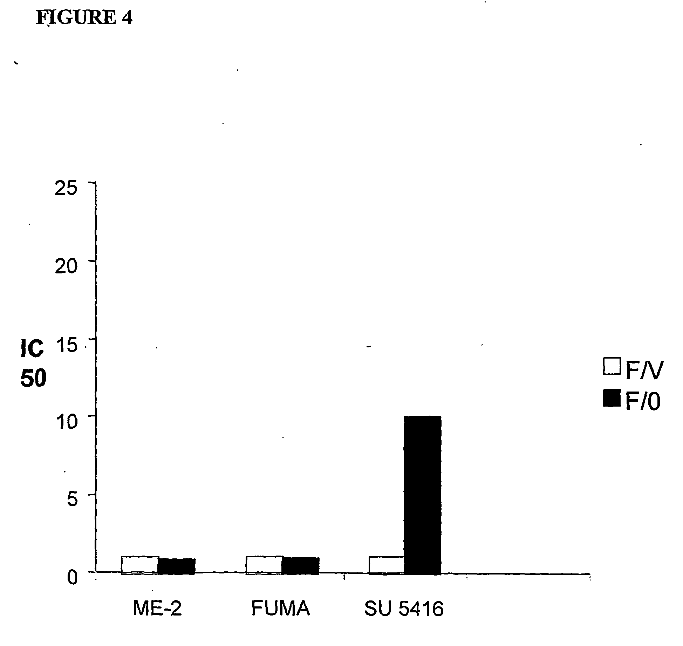

Selon un mode de réalisation avantageux de l'invention, les cellules endothéliales de phénotype angiogénique sont sensibles à un traitement anti-angiogénique inhibant la phosphorylation du VEGF-R2 (voir Figure 4).

L'expression "non protégées de l'apoptose" signifie que les cellules privées de sérum meurent au décours d'une cascade d'événements se traduisant par la fragmentation des noyaux. Ce phénomène est mis en évidence par comptage de noyaux cellulaires fragmentés selon la technique décrite par Gavrieli (Gavrieli et al., 1992).

L'apoptose peut être induite par exemple par la privation de sérum, ou par tout autre moyen connu de l'homme de l'art.

Le terme "traitement angiogénique" désigne la mise en présence des cellules avec une substance déclenchant un effet angiogénique, notamment le VEGF.

On vérifie que les cellules endothéliales de phénotype angiogénique sont sensibles à un traitement angiogénique, par le test de prolifération.

On vérifie que les cellules endothéliales de phénotype angiogénique sont protégées de l'apoptose par le VEGF, par le test de Gavrieli.

La présente invention concerne des cellules endothéliales telles que définies ci-dessus, caractérisées en ce que ce sont des cellules endothéliales de vaisseau, notamment des cellules endothéliales d'aorte, de cortex surrénal, de peau, de cerveau, de veine ou d'artère de cordon ombilical.

La présente invention concerne également un procédé de préparation de cellules endothéliales de phénotype angiogénique telles que définies ci-dessus, caractérisé en ce qu'il comprend les étapes suivantes :
- l'incubation de cellules endothéliales, notamment prélevées d'une aorte dans un milieu contenant de l'oestradiol et un facteur de croissance, notamment le VEGF, afin d'obtenir des clones de cellules endothéliales de phénotype angiogénique,
- le prélèvement d'un clone à l'aide d'une micropipette, parmi les clones susmentionnés, de cellules endothéliales de phénotype angiogénique, et la culture de ce clone dans un milieu contenant de l'oestradiol et du VEGF jusqu'à l'obtention de la confluence des cellules,
- la sélection des cellules endothéliales de phénotype angiogénique, par vérification du phénotype des cellules obtenues à l'étape précédente, à l'aide de tests de prolifération, de migration ou d'angiogenèse *in vitro.*

L'expression "clones de cellules endothéliales de phénotype angiogénique" désigne un ensemble de cellules répondant aux critères requis.

Après l'étape d'incubation, on prélève tous les clones, on les teste par angiogenèse *in vitro* et on sélectionne ceux qui sont "angiogéniques", c'est-à-dire ceux qui forment des tubes sous l'action du VEGF.

La présente invention concerne également un anticorps monoclonal dirigé contre les cellules endothéliales de phénotype angiogénique telles que définies ci-dessus, et inhibant l'angiogenèse, susceptible d'être obtenu par le procédé mentionné ci-après.

Par "anticorps capable d'inhiber l'angiogenèse" on désigne un anticorps doué d'activité anti-angiogénique comme sus définie.

Selon un mode de réalisation avantageux de la présente invention, un anticorps monoclonal dirigé contre des cellules endothéliales de phénotype angiogénique de l'invention présente les caractéristiques suivantes :
- il se lie à la surface des cellules endothéliales de phénotype angiogénique, et
- il reconnaît un motif présent exclusivement sur les cellules endothéliales de phénotype angiogénique, notamment un récepteur membranaire.

On vérifie que l'anticorps monoclonal dirigé contre des cellules endothéliales de phénotype angiogénique de l'invention se lie à la surface des cellules endothéliales de phénotype angiogénique par le test ELISA sur cellules ou de cytométrie, tels que définis dans les exemples.

On vérifie que l'anticorps monoclonal dirigé contre des cellules endothéliales de phénotype angiogénique de l'invention reconnaît un motif présent exclusivement sur les cellules endothéliales de phénotype angiogénique, notamment un récepteur membranaire par les mêmes tests que ceux indiqués ci-dessus.

La présente invention concerne également un procédé de préparation d'un anticorps monoclonal dirigé contre les cellules angiogéniques tel que défini ci-dessus, et inhibant l'angiogenèse, caractérisé en ce qu'il comprend les étapes suivantes :
- l'immunisation d'un animal par injection de cellules de phénotype angiogénique telles que définies ci-dessus,
- la fusion entre des myélomes d'un animal et des splénocytes d'un animal afin d'obtenir des hybridomes,
- la mise en culture des hybridomes ainsi obtenus,
- le clonage d'hybridomes, choisis parmi ceux obtenus à l'étape précédente et sécrétant des anticorps contre les cellules de phénotype angiogénique,
- la vérification des propriétés d'inhibition de l'angiogenèse des anticorps susmentionnés vis-à-vis des cellules angiogéniques, notamment à l'aide du test de prolifération et/ou de migration et/ou d'angiogenèse *in vitro.*

L'animal utilisé pour l'étape d'immunisation est notamment une souris ou un rat.

Les myélomes utilisés pour la fusion proviennent notamment d'une souris ou d'un rat.

Les splénocytes utilisés pour la fusion proviennent d'un animal de la même espèce que celle dont provient les myélomes, à savoir notamment d'une souris ou d'un rat.

On choisit les hybridomes qui sécrètent les anticorps contre les cellules de phénotype angiogénique par ELISA, ou par cytométrie, et on met en évidence la fonction anti-angiogénique par un test de prolifération et d'angiogenèse *in vitro.*

La présente invention concerne également des anticorps anti-idiotypiques dirigés contre des anticorps monoclonaux tels que définis ci-dessus, caractérisés en ce qu'ils sont capables d'inhiber les fonctions exercées (inhibition de l'angiogenèse) par les dits anticorps

Ces anticorps miment donc les structures des récepteurs et peuvent être considérés comme des récepteurs circulants, donc fonctionnant comme des anticorps anti-ligands. L'avantage réside en la construction d'anticorps anti ligands sans que la nature du ligand soit connue.

La présente invention concerne également des fragments Fab des anticorps monoclonaux, tels que définis ci-dessus, lesdits fragments étant capables d'inhiber l'angiogenèse.

Les fragments Fab peuvent être obtenus par clivage de la chaîne lourde des anticorps, notamment par la papaïne. Ils se lient donc aux récepteurs de la même façon mais ne les dimérisent pas, donc ils exercent uniquement une activité de blocage du récepteur.

La présente invention concerne un procédé de préparation des anticorps anti-idiotypiques tels que définis ci-dessus caractérisé en ce qu'il comprend les étapes suivantes :
- l'immunisation d'un animal par injection d'anticorps monoclonaux tels que définis ci-dessus, à savoir des anticorps anti-cellules angiogéniques,
- la fusion entre des myélomes d'un animal et des splénocytes d'un animal, afin d'obtenir des hybridomes,
- la mise en culture des hybridomes ainsi obtenus,
- le clonage d'hybridomes, choisis parmi ceux obtenus à l'étape précédente et sécrétant des anticorps dirigés contre les anticorps monoclonaux susmentionnés, utilisés pour l'immunisation, lesdits anticorps monoclonaux étant dirigés contre les cellules de phénotype angiogénique, et
- la vérification des propriétés d'inhibition des anticorps susmentionnés vis-à-vis de la fonction d'activation ou d'inhibition de l'angiogenèse des anticorps tels que définis ci-dessus, notamment à l'aide du test de prolifération et/ou de migration et/ou d'angiogenèse *in vitro.*

L'animal utilisé pour l'étape d'immunisation est notamment une souris ou un rat.

Les myélomes utilisés pour la fusion proviennent notamment d'une souris ou d'un rat.

Les splénocytes utilisés pour la fusion proviennent d'un animal de la même espèce que celle dont provient les myélomes, à savoir notamment d'une souris ou d'un rat.

On choisit les hybridomes qui sécrètent des anticorps dirigés contre les anticorps monoclonaux utilisés pour l'immunisation par ELISA en utilisant les fragments Fab des anticorps ayant servi d'immunogène comme substrat de la réaction.

La présente invention concerne également des anticorps anti-anti-idiotypiques dirigés contre des cellules endothéliales de phénotype angiogénique telles que définies ci-dessus, susceptibles d'être obtenus selon le procédé mentionné ci-après caractérisés en ce qu'ils sont capables d'inhiber l'angiogenèse. Ces anticorps étant dirigés contre un anticorps mimant un récepteur, ils se comportent comme des mimes du ligand. L'avantage est donc d'obtenir des mimes du ligand, même en ne connaissant pas sa structure.

La présente invention concerne également un procédé de préparation des anticorps anti-anti-idiotypiques capable d'inhiber l'angiogenèse dirigés contre des cellules endothéliales de phénotype angioginique telles que définies ci-dessus caractérisé en ce qu'il comprend les étapes suivantes :
- l'immunisation d'un animal par injection d'anticorps anti-idiotypiques tels que définis ci-dessus, à savoir des anticorps anti-anticorps anti-cellules angiogéniques,
- la fusion entre des myélomes d'un animal et des splénocytes d'un animal, afin d'obtenir des hybridomes,
- la mise en culture des hybridomes ainsi obtenus,
- le clonage d'hybridomes, choisis parmi ceux obtenus à l'étape précédente et sécrétant des anticorps anti-anti-idiotypiques dirigés contre les cellules de phénotype angiogénique, et
- la vérification des propriétés d'inhibition ou d'activation de l'angiogenèse des anticorps anti-anti-idiotypiques susmentionnés, notamment à l'aide du test de prolifération et/ou de migration et/ou d'angiogenèse *in vitro.*

L'animal utilisé pour l'étape d'immunisation est notamment une souris ou un rat.

Les myélomes utilisés pour la fusion proviennent notamment d'une souris ou d'un rat.

Les splénocytes utilisés pour la fusion proviennent d'un animal de la même espèce que celle dont provient les myélomes, à savoir notamment d'une souris ou d'un rat.

On choisit les hybridomes qui sécrètent des anticorps anti-anti-idiotypiques dirigés contre les cellules de phénotype angiogénique par mesure de leur liaison aux fragments Fab de l'anticorps anti-idiotypique.

La présente invention concerne également une composition pharmaceutique, caractérisée en ce qu'elle contient, à titre de substance active, un inhibiteur de l'angiogenèse, choisi parmi un anticorps tel que défini ci-dessus, un fragment Fab tel que défini ci-dessus, en association avec un vecteur pharmaceutiquement acceptable, ladite composition pharmaceutique étant susceptible d'être administrée à raison d'environ 0,01 à environ 20 mg/kg/injection.

La présente invention concerne également une composition vaccinale, caractérisée en ce qu'elle comprend, à titre de substance active un anticorps monoclonal tel que défini ci-dessus, ou des fragments Fab tels que définis ci-dessus, ou un anticorps anti-anti-idiotypique tel que défini ci-dessus, en association avec un adjuvant pharmaceutiquement acceptable.

La présente invention concerne également l'utilisation d'un inhibiteur de l'angiogenèse, choisi parmi un anticorps tel que défini ci-dessus, un fragment Fab tel que défini ci-dessus, ou d'un anticorps anti-anti-idiotypique tel que défini ci-dessus, pour la préparation d'un médicament destiné au traitement de pathologies nécessitant l'inhibition de la prolifération endothéliale, notamment dans le cadre des pathologies suivantes : les cancers, la dégénérescence musculaire liée à l'âge, les rétinopathies diabétiques, la polyarthrite rhumatoïde, les angiomes, les angiosarcomes, en particulier la maladie de Castelman et le sarcome de Kaposi.

La présente invention concerne également l'utilisation d'un inhibiteur de l'angiogenèse, choisi parmi un anticorps tel que défini ci-dessus, un fragment Fab tel que défini ci-dessus, ou d'un anticorps anti-anti-idiotypique tel que défini ci-dessus, pour la préparation d'un médicament destiné au traitement de pathologies nécessitant l'inhibition de l'activation endothéliale, notamment dans le cadre des pathologies suivantes : le rejet d'allogreffe et de xénogreffe, les acrocyanoses, les sclérodermies, ou dans le cadre de la préparation de greffons entre le prélèvement et la transplantation.

L'expression "l'inhibition de l'activation endothéliale" peut être déterminée par un test mesurant la présence à la surface cellulaire d'un marqueur d'activation (notamment ICAM-1, V-CAM).

### DESCRIPTION DES FIGURES

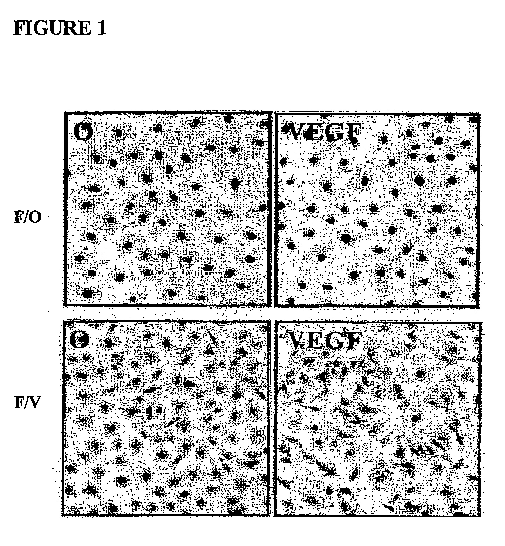

Les Figures 1A, 1B, 1C et 1D représentent la morphologie des deux phénotypes cellulaires en culture, à savoir les cellules endothéliales d'aorte de foetus de boeuf non angiogéniques (F/0) et les cellules endothéliales d'aorte de foetus de boeuf angiogéniques (F/V).
   La Figure 1A correspond à des cellules F/0 cultivées en absence de VEGF
   La Figure 1B correspond à des cellules F/0 cultivées en présence de VEGF
   La Figure 1C correspond à des cellules F/V cultivées en absence de VEGF
   La Figure 1D correspond à des cellules F/V cultivées en présence de VEGF

On constate que le VEGF maintient des phénotypes angiogéniques distincts. Seules les cellules F/V changent de morphologie sous l'action du VEGF. Les cellules F/O sont arrondies et plus grosses que les cellules F/V. L'ajout de VEGF ne modifie pas la morphologie des cellules F/O. Les cellules F/V cultivées sans VEGF ont une morphologie proche de celle des cellules F/O. En présence de VEGF, elles apparaissent étirées.

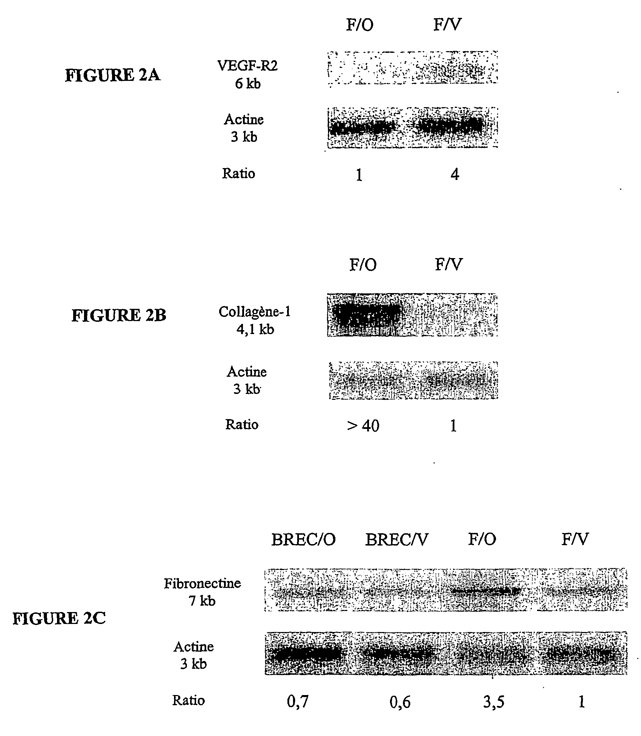

Les Figures 2A, 2B et 2C représentent l'analyse de l'expression génique par transfert d'ARN (Northern blot).
   La Figure 2A correspond à la comparaison par transfert d'ARN de l'expression de VEGF-R2 (récepteur 2 du VEGF) dans les cellules F/0 et F/V. On utilise comme témoin l'actine, dont l'expression ne varie pas dans les cellules F/0 et F/V. Cette Figure démontre que l'expression de VEGF-R2 est fortement augmentée dans les cellules F/V par rapport aux cellules F/0.
   La Figure 2B correspond à la comparaison par transfert d'ARN de l'expression du collagène-1 dans les cellules F/0 et F/V. On utilise comme témoin l'actine, dont l'expression ne varie pas dans les cellules. Cette Figure démontre que l'expression du collagène-1 est supérieure dans les cellules F/0 par rapport à celle dans les cellules F/V.
   La Figure 2C correspond à la comparaison par transfert d'ARN de l'expression de la fibronectine dans les cellules F/0, F/V, BREC/0 (cellules endothéliales non angiogénique de rétine) et BREC/V (cellules endothéliales angiogéniques de rétine)(Hutchings et al., 2002). On utilise comme témoin l'actine, dont l'expression ne varie pas dans les cellules. Cette Figure démontre que l'expression de la fibronectine est supérieure dans les cellules F/0 par rapport à celle dans les cellules F/V. De même que pour le VEGF-R2, il n'y a pas de différence significative d'expression de la fibronectine dans les cellules BREC/V et BREC/0.

Dans toutes ces figures, la ligne "ratio" correspond au rapport entre l'expression du gène d'intérêt dans les deux types cellulaires.

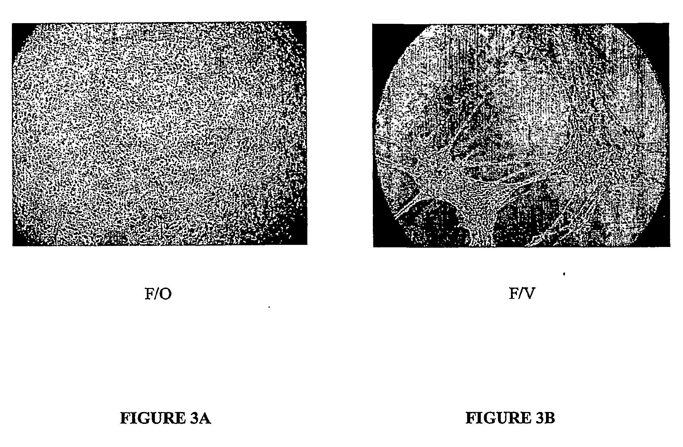

Les Figures 3A et 3B représentent l'angiogenèse *in vitro* sur Matrigel (Becton Dickinson).
   La Figure 3A représente l'absence de formation de tubules par les cellules F/O. Les cellules F/O demeurent en une monocouche régulière sur Matrigel.
   La Figure 3B représente la formation de tubules par les cellules F/V. Les cellules F/V se différencient sur Matrigel, elles s'alignent bout à bout pour reconstituer des pseudo-capillaires.
La Figure 4 représente la sensibilité des cellules à des agents anti-angiogéniques particuliers, que sont : ME-2, FUMA et SU5416.

Les colonnes blanches correspondent aux cellules endothéliales non angiogéniques F/0 et les colonnes noires correspondent aux cellules endothéliales angiogéniques F/V.

"ME-2" correspond au méthoxyestradiol ; "FUMA" correspond à la fumagilline ; "SU 5416" est un inhibiteur de l'activité kinase du VEGF-R2.

Les cellules F/0 et F/V sont identiquement sensibles à la fumagilline (FUMA) et au méthoxyestradiol (ME-2). En revanche, les cellules F/V sont beaucoup plus sensibles que les cellules F/0 à l'agent anti-angiogénique interférant avec le VEGF-R2 (SU 5416).

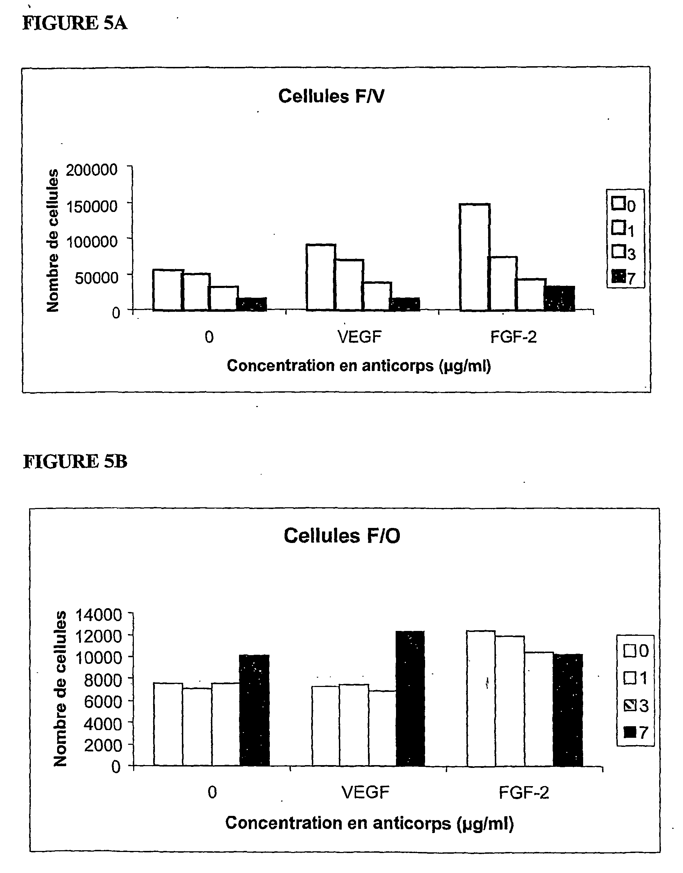

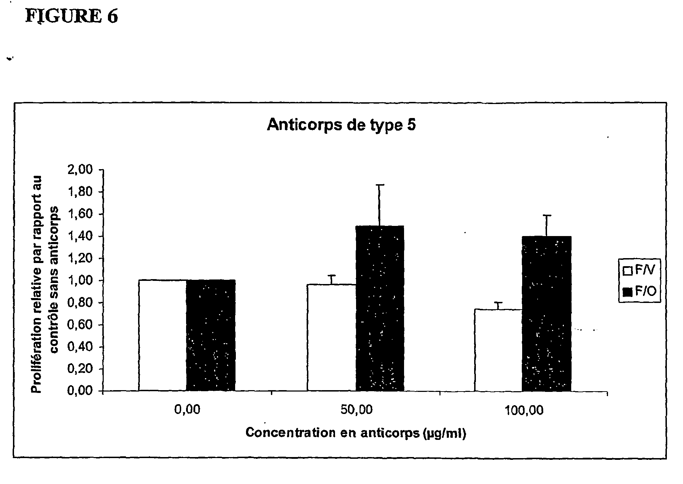

Les Figures 5A, 5B et 6 illustrent les fonctions mitogènes des anticorps monoclonaux dirigés contre les cellules endothéliales angiogéniques. Certains anticorps sont anti-angiogéniques, c'est à dire capables d'inhiber de façon spécifique la prolifération des seules cellules endothéliales angiogéniques (appelés anticorps de type 3). D'autres anticorps ont une activité pro-angiogénique vis-à-vis des cellules endothéliales (appelés anticorps de type 5).
Les Figures 5A et 5B montrent l'action anti-angiogénique des anticorps de type 3. La Figure 5A concerne en particulier l'action des anticorps de type 3 sur la prolifération des cellules F/V de phénotype angiogénique. La Figure 5B concerne l'action des anticorps de type 3 sur la prolifération des cellules non angiogéniques F/O. Le test effectué est un test de prolifération comme décrit dans les exemples. Les cellules sont cultivées dans du DMEM supplémenté de 5 % de sérum de veau nouveau-né auquel est ajouté soit aucun facteur angiogénique (condition 0), soit 2 ng/ml de VEGF (condition VEGF), soit 2 ng/ml de FGF-2 (condition FGF-2). L'anticorps est ajouté en même temps que les facteurs angiogéniques, à des concentrations variables (0, 1, 3 ou 7 µg/ml).

Les colonnes blanches correspondent à la prolifération des cellules sans anticorps. Les colonnes en pointillés correspondent à la prolifération des cellules en présence de 1 µg/ml d'anticorps. Les colonnes hachurées correspondent à la prolifération des cellules en présence de 3 µg/ml d'anticorps. Les colonnes noires correspondent à la prolifération des cellules en présence de 7 µg/ml d'anticorps.

On constate que les anticorps de type 3 inhibent de façon dose-dépendante la prolifération des cellules F/V sans affecter celle des cellules F/0.

On constate que les anticorps de type 3 inhibent l'effet mitogène du VEGF et du FGF-2 vis-à-vis des cellules endothéliales angiogéniques.
La Figure 6 montre l'action pro-angiogénique des anticorps de type 5. Le test effectué est un test de prolifération comme décrit dans les exemples. Les cellules sont cultivées dans du DMEM supplémenté de 5 % de sérum de veau nouveau-né. L'anticorps est ajouté en même temps que les facteurs angiogéniques, à des concentrations variables (0, 50 ou 100 µg/ml).

Les colonnes blanches correspondent à la prolifération relative des cellules F/O en présence de concentrations variables d'anticorps de type 5. Les colonnes noires correspondent à la prolifération relative des cellules F/V en présence de concentrations variables d'anticorps de type 5.

On constate que les anticorps de type 5 stimulent la prolifération des cellules F/O. En revanche, ils n'ont pas d'effet sur la prolifération des cellules F/V.

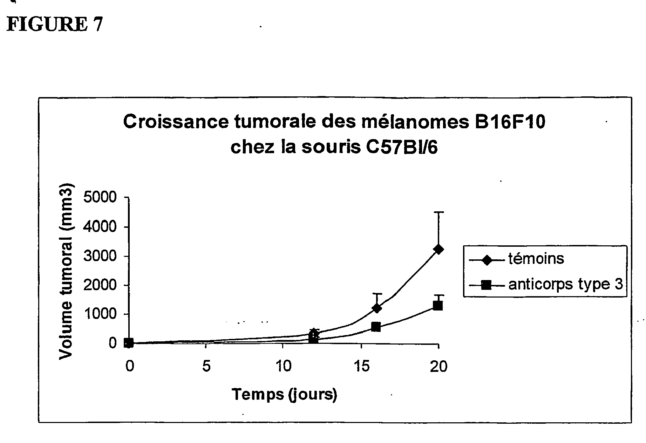

La Figure 7 représente la fonction des anticorps de type 3 en tumorigenèse.

La courbe avec les losanges noirs correspond aux souris non, traitées et la courbe avec les carrés noirs correspond aux anticorps de type 3.

Cette Figure représente le volume tumoral moyen (6 souris par groupe) en mm³ en fonction du temps (en jours).

On constate que les anticorps de type 3 sont capables d'inhiber de façon significative le développement de mélanomes B16F10 chez la souris C57BL/6.

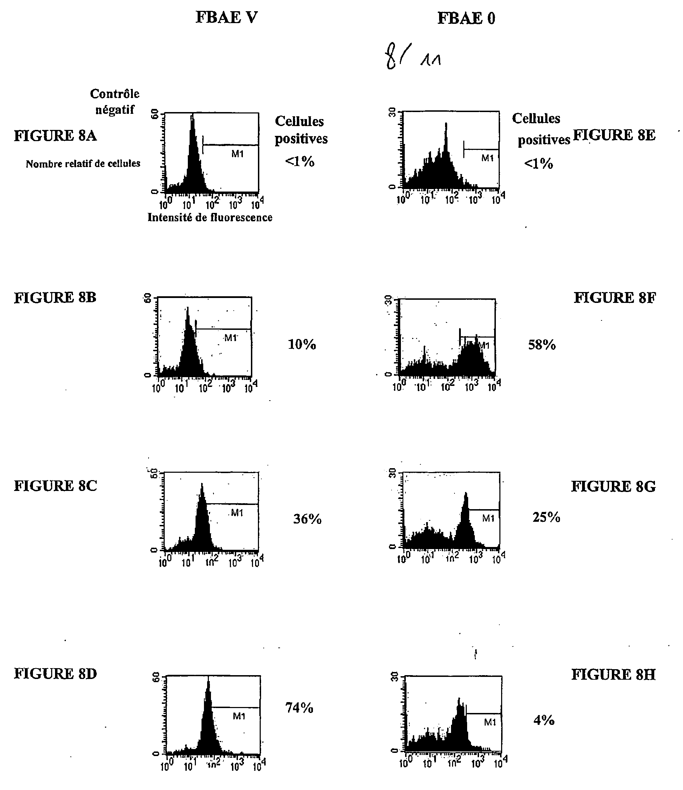

Les Figures 8A à 8H représentent des analyses par cytométrie de flux pour des cellules F/V (FBAE V)(Figures 8A à 8D) et pour des cellules F/0 (FBAE 0)(Figures 8E à 8H), incubées avec différents anticorps . Les Figures 8A et 8E correspondent au contrôle négatif ; les Figures 8B et 8F à l'anticorps A défini ci- après ; les Figures 8C et 8B à l'anticorps B défini ci-après; les Figures 8D et 8H à l'anticorps C défini ci-après.

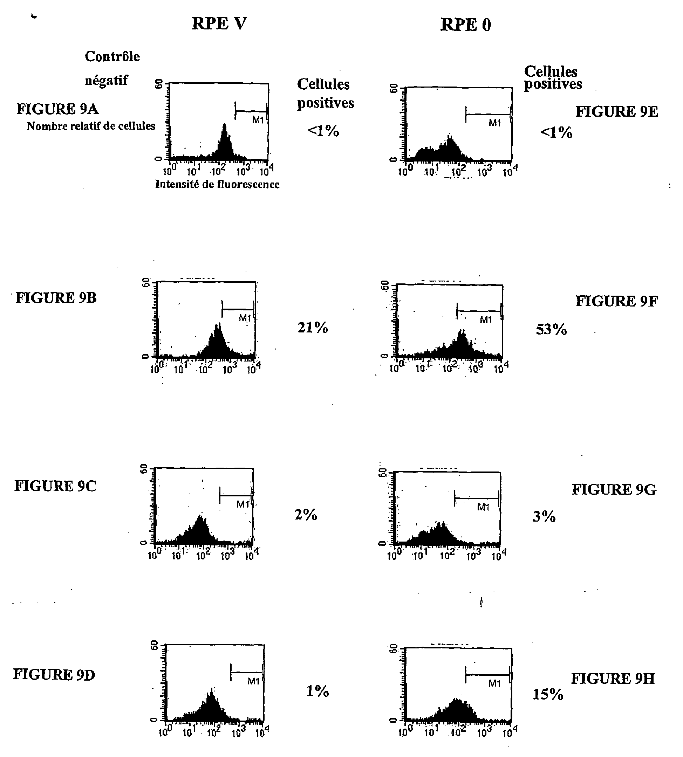

Les Figures 9A à 9H représentent des analyses par cytométrie de flux pour des cellules RPE V(Figures 9A à 9D) et pour des cellules RPE 0 (Figures 9E à 9H), incubées avec différents anticorps. Les Figures 9A et 9E correspondent au contrôle négatif; les Figures 9B et 9F à l'anticorps A défini ci-après; les Figures 9C et 9G à l'anticorps B défini ci-après; les Figures 9D et 9H à l'anticorps C défini ci-après.

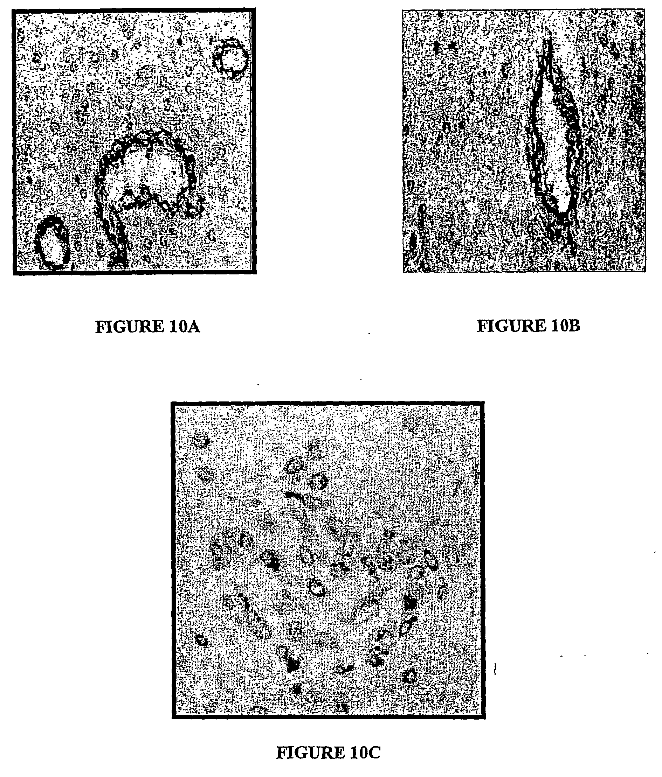

Les Figures 10A, 10B et 10C correspondent à des résultats d'immuno-histochimie de coupes de glioblastomes. La Figure 10A correspond à un glioblastome incubé avec un anticorps reconnaissant les cellules musculaires lisses des vaisseaux ("SM actin"). La Figure 10B correspond à un glioblastome incubé avec un anticorps commercial reconnaissant les cellules endothéliales (aticorps anti-CD 31)(DAKO). La Figure 10C correspond à un glioblastome incubé avec un anticorps reconnaissant l'anticorps C défini ci-après.

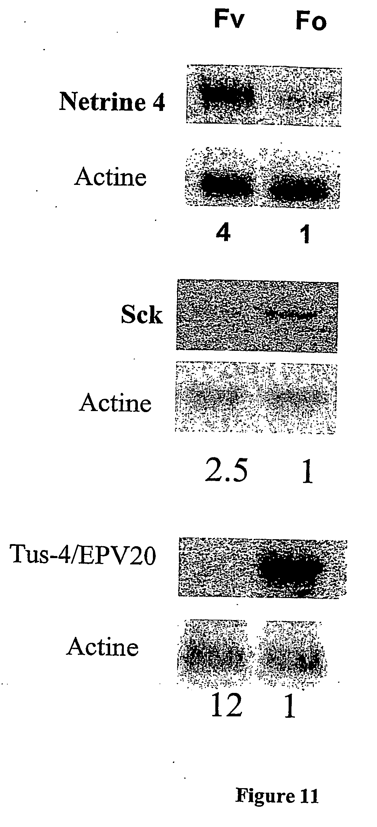

La Figure 11 correspond à un transfert d'ARN, en utilisant les sondes correspondant au fragment 74/1268 de la séquence AF063658 (Genbank) pour le VEGF-R2, au fragment 4155/4624 de la séquence AB008683 (Genbank) pour le collagène-1 et au fragment 380/847 de la séquence M10905 (Genbank) pour la fibronectine, à la séquence AK024691 de la netrine 4, la séquence AK 025662 pour la Sck, la séquence X85799 pour le Tus-4/EPV20/.

### MATERIEL ET METHODES

### 1. Etablissement des clones de différents phénotypes

Les cellules endothéliales d'aorte de foetus de boeuf (FBAE ou F) sont prélevées mécaniquement à partir de la lumière aortique à l'aide d'un grattoir et incubées sur des boîtes de culture préalablement tapissées de gélatine ou collagène I (10 µg/ml) préparé selon le paragraphe "angiogenèse *in vitro"* (0,2% en PBS, 24 h à 4°C) dans du milieu DMEM contenant 10% de sérum de veau nouveau-né (SVNN). Les boîtes sont soit supplémentées d'oestradiol (10-9 M) et de 1 ng/ml de VEGF (F/V) ou cultivées sans oestrogène et sans VEGF (F/0). Le milieu est change après 4 jours. Entre 6 et 10 jours, les clones de chacune des cultures sont isolés. Pour cela, les clones visualisés au microscope comme des foyers de 100 à 1000 cellules sont aspirés à l'aide d'une micropipette et transférés dans des puits de 2 cm². Chacun des clones est cultivé dans le milieu correspondant (oestradiol + VEGF pour les cellules F/V ; sans oestradiol et sans VEGF pour les cellules F/0) puis une fois que la confluence est obtenue (environ 6 jours pour les F/V, 15 jours pour les F/0) les monocouches de cellules sont trypsinisées et réensemencées dans des boîtes de 5 cm de diamètre. Une fois la confluence obtenue, les cellules sont encore trypsinisées, une fraction aliquote est congelée, puis la validation (détermination de la réponse au VEGF dans l'un des tests suivants : migration, prolifération, angiogenèse) du clone est initiée.

### Facteurs utilisés

Le VEGF (isoforme de 165 acides aminés) est produit par infection de cellules d'insecte SF9 par un baculovirus recombinant contenant l'ADNc correspondant (Plouët et al., 1997).

### 2. Techniques d'étude in vitro : validation des clones

### Migration

La technique a été décrite précédemment par Jonca et al. (1997).

### Prolifération

Des plaques de 12 puits sont ensemencées à faible densité (5000 à 10000 cellules/puits), dans du milieu DMEM à 8% de sérum. Après 24 heures, la prolifération des cellules est stimulée par ajout d'une gamme de facteur de croissance, de médicaments anti-angiogéniques (méthoxyestradiol, fumagilline, SU5416 ciblée sur le VEGF-R2 - Plouët et al., 2001) ou d'anticorps de la présente invention. Les cellules sont restimulées après 48 heures et puis après un total de cinq jours en culture les cellules sont trypsinisées et comptées avec un compteur Coulter.

### Angiogenèse in vitro

Quatre queues de rat ont été dépiautées et disséquées pour récupérer les faisceaux blancs qui sont constitués en majeure partie de collagène de type I. Le collagène est extrait de ces fibres dans 50 ml d'acide acétique 0,5 M froid et agités sur une nuit. Le liquide est ensuite centrifugé à 5000g pendant 40 minutes et le surnageant est récupéré. L'extraction est refaite une fois avec 20 ml d'acide acétique, les surnageants sont mélangés et puis dialysés contre 1 l d'acide acétique 0,2 M. La concentration en collagène est ajustée à 3 mg/ml par pesée.

La préparation des gels pour l'angiogenèse *in vitro* s'effectue sur de la glace pour conserver la solution de collagène sous forme liquide. Un ml de collagène (5 mg/ml) est mélangé à 0,5 ml de DMEM 10X (contenant une concentration 10X en antibiotiques et en glutamine), 0,9 ml de H₂O stérile et 0,1 ml de bicarbonate de sodium 1M. Une fois le pH ajusté à 7,4, il est ajouté un volume égal de matrigel (Becton Dickinson).

Le gel est coulé dans des puits de culture (2 mm d'épaisseur) et incubé à 37°C pour se solidifier. Les cellules sont rajoutées après 15 minutes (100 000 cellules/cm²) sur la surface du gel. Après 2 heures, les différents facteurs solubles sont ajoutés et les cellules sont observées et photographiées après 24 heures.

### Transfert d'ARN (Northern blot)

Les cellules F/0 ou F/V sont cultivées en milieu DMEM contenant 10% de SVNN jusqu'à la demi-confluence. Les cellules sont ensuite trypsinisées et centrifugées. Les ARN sont extraits à l'aide d'une solution de TRIZOL (Invitrogen). 2µg d'ARN poly A+ sont séparés par électrophorèse en gel d'agarose à 1,2% (p/vol) en conditions dénaturantes, transférés sur membrane de nylon (Hybond N Amersham) et fixés sous UV (0,5 J/cm2) selon la technique décrite par Sambrook et ses collaborateurs en 1989. L'hybridation est réalisée à 42°C dans le milieu réactionnel suivant : 50% formamide, 5X Denhardt's et 0,5% SDS (p/vol), contenant 0,2% d'ADN de sperme de hareng et 1ng/ml de sonde radioactive (activité spécifique comprise entre 10⁸ et 10⁹ cpm/µg) marquée au 32P à l'aide du kit Megaprime^{™} DNA labelling systems (Amersham). Les lavages sont réalisés à 45°C dans du SSPE 0,2X contenant 0,1% de SDS.

Les sondes utilisées correspondent au fragment 74/1268 de la séquence AF063658 (Genbank) pour le VEGF-R2, au fragment 4155/4624 de la séquence AB008683 (Genbank) pour le collagène-1 et au fragment 380/847 de la séquence M10905 (Genbank) pour la fibronectine, à la séquence AK024691 de la netrine 4, la séquence AK 025662 pour la Sck, la séquence X85799 pour le Tus-4/EPV20/

### 3. Production d'anticorps monoclonaux spécifiques du phénotype FBAE V

### Immunisation soustractive des souris par les cellules FBAE

Cinq souris Balbc de 6 semaines sont immunisées par une injection de 200 µl contenant 2 × 10⁶ cellules F/0. Vingt-quatre et quarante-huit heures après, les souris reçoivent 200 mg/kg de poids corporel de cyclophosphamide afin de détruire les lymphocytes activés et rendre ainsi les souris tolérantes à l'endothélium de phénotype non angiogénique. Quinze jours après, les souris reçoivent une injection de 2 × 10⁶ cellules F/V. L'opération est répétée au jour 30 et au jour 45. Les injections ont été effectuées par voie intrapéritonéale. L'évaluation de la réponse de chaque souris à l'immunisation est faite par cytométrie de flux à partir d'un prélèvement de sérum.

### Fusion

La technique utilisée pour la réalisation des hybridomes dérive de celle mise au point par Kohler et Milstein (1975).

La veille de la fusion, soit à J47, cinq souris Balbc non-immunisées sont sacrifiées pour l'obtention des macrophages servant de cellules nourricières aux hybridomes. Les macrophages sont récupérés par injection de 8 ml de milieu de culture ISCOVE dans le péritoine des souris. Le milieu est aspiré et refoulé 2 fois dans la cavité péritonéale puis récupéré et centrifugé. Après centrifugation, les macrophages sont repris dans du milieu ISCOVE contenant 20% de sérum de veau foetal (Biochrom, lot 5612). Ces cellules sont ensuite distribuées dans 20 plaques de 96 puits à raison de 100 µl par puits (environ 3 × 10⁴ cellules/puits).

Le lendemain, soit à J 48, les souris présentant les titres les plus élevés d'anticorps dirigés contre les cellules F/V sont sacrifiées. Leurs rates sont prélevées et dilacérées dans du milieu ISCOVE pour libérer les splénocytes. Le tissu conjonctif est dégagé et les splénocytes sont centrifugés et comptés. En parallèle, la lignée de myélome de souris Ag8X63 a été cultivée depuis 10 jours dans du milieu ISCOVE (Invitrogen) à 20% de sérum Myoclone (Invitrogen). Ces cellules sont lavées et numérées.

Les deux types cellulaires, splénocytes et myélomes, sont mélangés de façon à obtenir un rapport de 1 cellule de myélome Ag8X63 (Kearney et al., 1979) pour 6 splénocytes. La fusion s'effectue par ajout de 20 fois 50 µl de polyéthylène glycol (PEG) à 30 secondes d'intervalle. Quatre ml de milieu ISCOVE préchauffé à 37°C sont alors ajoutés goutte à goutte sur la suspension cellulaire, puis après une période d'incubation de 4 minutes à 37°C, 4 ml sont ajoutés. La suspension est centrifugée puis le culot cellulaire est alors repris dans 100 ml de milieu ISCOVE complémenté avec 20% de sérum de veau foetal et du milieu sélectif HAT 1X (50X : Hypoxanthine 5 mM, Aminoptérine 20 µM et Thymidine 0,8 mM) qui permet l'élimination des splénocytes et des cellules de myélome. La suspension est alors distribuée à raison de 100 µl par puits sur les macrophages (environ 100 000 cellules).

Après 5 jours, 100 µl de milieu HAT sont ajoutés, et entre 8 et 14 jours le milieu conditionné de chaque hybridome est prélevé pour mesurer les anticorps dirigés contre les cellules endothéliales de différents phénotypes par ELISA.

Les hybridomes sélectionnés par leur capacité à sécréter des anticorps dirigés contre les cellules F/0 ou F/V sont alors clonés (premier clonage), c'est-à-dire que les cellules sont ensemencées en condition de dilution limite (5 cellules/ml) sous un volume de 0,1 ml par puits. Le milieu (ISCOVE supplémenté de sérum et de HAT) est changé après 10 jours. Après 15 jours, certains puits contiennent des foyers de cellules qui se sont multipliées à partir de la cellule ensemencée au départ, donc toutes ces cellules sont identiques et sont issues du même clone. Quand la surface occupée par les cellules représente au moins la moitié de la surface totale du puits, le milieu est prélevé et analysé comme précédemment. A ce stade on peut sélectionner les clones producteurs d'anticorps et connaître leur spécificité pour les cellules F/0 ou F/V (deuxième clonage).

Une fois les clones identifiés, leur nature monoclonale a été affirmée par l'opération classique consistant à ensemencer une plaque de 96 puits avec des cellules issues du même clone diluées en conditions limites comme précédemment. Les clones sécréteurs doivent donc tous sécréter un anticorps de même spécificité vis-à-vis des cellules F/V par rapport aux cellules F/0 (criblage par ELISA) pour que l'on déclare cet anticorps monoclonal.

Un troisième clonage est alors effectué pour s'assurer que les clones sont bien monoclonaux dans des conditions identiques à celles indiquées ci-dessus.

### Méthode de criblage des clones

Le criblage du sérum prélevé et des surnageants des hybridomes s'est effectué par ELISA et cytométrie de flux.

### ELISA

15000 cellules F/0 ou F/V sont ensemencées par puits dans des plaques 96 puits (100 µl/puits) dans du milieu DMEM contenant 10% de sérum de veau nouveau-né. Après 36 h, les cellules sous-confluentes sont rincées 3 fois dans une solution de PBS contenant 0,005% de Tween 20. Les sites potentiels de liaison non spécifiques des anticorps sont saturés par incubation dans une solution de glycine 1 M, pH 8,1 (2h à température ambiante) suivie de 3 lavages (PBS/Tween 0,05%) et d'une incubation d'une nuit à 4°C dans une solution d'IgG irrelevantes à 10 µg/ml dans du PBS. La solution de blocage est éliminée, puis les anticorps à tester sont ajoutés. Après 2 h d'incubation à température ambiante, les cellules sont lavées 3 fois en PBS / Tween 0,05% pour éliminer les anticorps non fixés. Les complexes cellules-anticorps sont incubés pendant 1 heure en présence d'un anti-corps secondaire anti-souris couplé à la peroxydase (Amersham NA 931) dilué au 1/4000 dans du PBS contenant 0,05% de Tween et 0,5% de BSA. Après 3 lavages (PBS / Tween 0,05%), le substrat de la peroxydase (OPD Sigma) est ajouté à raison de 100 µl/puits. Après 10 à 30 min d'incubation à l'abri de la lumière, la réaction colorimétrique est arrêtée par ajout de 100 µl de H₂SO₄M. L'intensité du signal est mesuré par spectrophotométrie à 490 nm.

### Cytométrie

Le surnageant (ou le sérum) à tester est incubé avec des cellules F/0, F/V, RPE/0 ou des cellules RPE/V pendant 45 minutes. Les cellules sont ensuite lavées 3 fois dans du PBS-BSA 0,02%. Un deuxième anticorps anti-IgG de souris couplé à la fluorescéine isothiocyanate est ajouté sur les cellules (F-877, Sigma Immuno Chemicals). L'intensité de fluorescence des cellules est mesurée par cytométrie de flux et comparée entre les cellules F/0 et F/V. L'intensité de fluorescence est mesurée dans un cytomètre de flux ELITE.

### Isotypage

La caractérisation isotypique de chaque clone est mesurée à l'aide d'un test rapide d'isotypage des anticorps murins MABTEST (Adiatec).

### Tumorigenèse

200 000 cellules de mélanome B16 sont injectées à des souris C57. Huit jours après, quand les tumeurs ont donné naissance à des nodules palpables, les anticorps sont injectés 2 fois par semaine par voie intrapéritonéale à la dose de 50 µg dilué dans 200 µl de PBS. Le volume de la tumeur est mesuré 2 fois par semaine au pied à coulisse et exprimé par la formule V= ½ x L × 1² (L et 1 désignant respectivement la plus grande et la plus petite dimension).

### 4. Production d'anticorps anti-idiotypiques

On injecte par voie sous-cutanée à des souris 10 µg d'un anticorps monoclonal dirigé contre les cellules endothéliales non angiogéniques, ledit anticorps monoclonal étant obtenu selon le procédé décrit dans le paragraphe précédent, en association avec 100 µl d'adjuvant de Freund (Sigma), l'anticorps étant purifié selon des méthodes standard par précipitation au sulfate d'ammonium puis par chromatographie d'échange d'ions.

L'injection est répétée 15, 30 et 45 jours après.

Cinquante-cinq jours après la première injection, on injecte à des souris 10 µg du même anticorps par voie intrapéritonéale. Cinquante-huit jours après, les souris sont sacrifiées et leurs rates sont prélevées et dilacérées dans du milieu ISCOVE pour libérer les splénocytes. Les splénocytes sont fusionnés avec des cellules de myélome de souris, notamment des cellules AG8X 63 (Kearney et al., 1979) et incubés à raison de 100 000 cellules/puits.

La fusion s'effectue par ajout de 20 fois 50 µl de polyéthylène glycol (PEG) à 30 secondes d'intervalle. Quatre ml de milieu ISCOVE préchauffé à 37°C sont alors ajoutés goutte à goutte sur la suspension cellulaire, puis après une période d'incubation de 4 minutes à 37°C, 4 ml sont ajoutés. La suspension est centrifugée puis le culot cellulaire est alors repris dans 100 ml de milieu ISCOVE complémenté avec 20% de sérum de veau foetal et du HAT 1X (50X : Hypoxanthine 5 mM, Aminoptérine 20 µM et Thymidine 0,8 mM) et distribuées à raison de 100 µl par puits sur les macrophages.

Après 5 jours, 100 µl de milieu HAT sont ajoutés, et entre 8 et 14 jours le milieu conditionné de chaque hybridome est prélevé pour mesurer les anticorps dirigés contre les anticorps ayant servi d'agents immunogènes, c'est à dire dirigés contre des cellules endothéliales.

Les hybridomes sélectionnés par leur capacité à sécréter des anticorps dirigés contre des anticorps monoclonaux dirigés eux-mêmes contre des cellules F/0 ou F/V sont alors clonés (premier clonage), c'est-à-dire que les cellules sont ensemencées en condition de dilution limite (5 cellules/ml) sous un volume de 0,1 ml par puits. Le milieu de culture HAT est changé après 10 jours. Après 15 jours, certains puits contiennent des foyers de cellules qui se sont multipliées à partir de la cellule ensemencée au départ, donc toutes ces cellules sont identiques et sont issues du même clone. Quand la surface occupée par les cellules représente au moins la moitié de la surface totale du puits, le milieu est prélevé et analysé comme précédemment par ELISA. A ce stade on peut sélectionner les clones producteurs d'anticorps et connaître leur spécificité pour les anticorps monoclonaux dirigés contre les cellules F/0 ou F/V (deuxième clonage).

Une fois les clones identifiés, leur nature monoclonale est affirmée par l'opération classique consistant à ensemencer une plaque de 96 puits avec des cellules issues du même clone diluées en conditions limites comme précédemment. Les clones sécréteurs doivent donc tous sécréter un anticorps de même spécificité pour que l'on déclare cet anticorps monoclonal.

Un troisième clonage est alors effectué pour s'assurer que les clones sont bien monoclonaux dans les conditions identiques à celles définies ci-dessus.

Les anticorps anti-idiotypiques sont criblés par une batterie de tests, notamment par un test ELISA anti-idiotypique : les anticorps dirigés contre les cellules endothéliales de l'invention sont tapissés dans du tampon carbonate 50 mM pH 9 à une dilution de 10 µg/ml. La surface des puits est ensuite incubée avec de la sérumalbumine à 0,5%. Les surnageants d'hybridomes sont ensuite incubés dans les boîtes et leur présence est révélée par les anticorps anti-immunoglobulines de souris conjugués à la peroxydase.

On vérifie également que les anticorps anti-idiotypiques inhibent la fonction des anticorps monoclonaux contre lesquels ils sont dirigés vis-à-vis des cellules endothéliales, notamment qu'ils inhibent la prolifération des cellules F/V incubées avec ng/ml et 20 µg/ml d'anticorps monoclonal anti-cellules endothéliales angiogéniques ou non.

### 5. Production d'anticorps anti-anti-idiotypiques

On injecte par voie sous-cutanée à des souris 10 µg d'un anticorps anti-idiotypique, tel qu'obtenu selon le procédé décrit dans le paragraphe précédent, en association avec 100 µl d'adjuvant de Freund.

L'injection est répétée 15, 30 et 45 jours après.

Cinquante-cinq jours après la première injection, on injecte à des souris 10 mg d'anticorps anti-idiotypiques par voie intrapéritonéale. Cinquante-huit jours après, les souris sont sacrifiées et leurs rates sont prélevées et dilacérées dans du milieu ISCOVE pour libérer les splénocytes. Les splénocytes sont fusionnés avec des cellules de myélome de souris, notamment des cellules AG8X 63, et incubés à raison de 100 000 cellules/puits.

La fusion s'effectue par ajout de 20 fois 50 µl de polyéthylène glycol (PEG) à 30 secondes d'intervalle. Quatre ml de milieu ISCOVE préchauffé à 37°C sont alors ajoutés goutte à goutte sur la suspension cellulaire, puis après une période d'incubation de 4 minutes à 37°C, 4 ml sont ajoutés. La suspension est centrifugée puis le culot cellulaire est alors repris dans 100 ml de milieu ISCOVE complémenté avec 20% de sérum de veau foetal et du HAT 1X (50X : Hypoxanthine 5 mM, Aminoptérine 20 µM et Thymidine 0,8 mM) et distribuées à raison de 100 µl par puits sur les macrophages.

Après 5 jours, 100 µl de milieu HAT sont ajoutés, et entre 8 et 14 jours le milieu conditionné de chaque hybridome est prélevé pour mesurer les anticorps dirigés contre les anticorps anti-idiotypiques.

Les hybridomes sélectionnés par leur capacité à sécréter des anticorps dirigés contre des anticorps anti-idiotypiques sont alors clonés (premier clonage), c'est-à-dire que les cellules sont ensemencées en condition de dilution limite (5 cellules/ml) sous un volume de 0,1 ml par puits. Le milieu de culture ISCOVE contenant 20% de sérum et de HAT est changé après 10 jours. Après 15 jours, certains puits contiennent des foyers de cellules qui se sont multipliées à partir de la cellule ensemencée au départ, donc toutes ces cellules sont identiques et sont issues du même clone. Quand la surface occupée par les cellules représente au moins la moitié de la surface totale du puits, le milieu est prélevé et analysé comme précédemment par ELISA. A ce stade on peut sélectionner les clones producteurs d'anticorps et connaître leur spécificité pour les anticorps anti-idiotypiques (deuxième clonage).

Une fois les clones identifiés, leur nature monoclonale est affirmée par l'opération classique consistant à ensemencer une plaque de 96 puits avec des cellules issues du même clone diluées en conditions limites comme précédemment. Les clones sécréteurs doivent donc tous sécréter un anticorps de même spécificité pour que l'on déclare cet anticorps monoclonal.

Un troisième clonage est alors effectué pour s'assurer que les clones sont bien monoclonaux identiques à celles décrites ci-dessus.

Les anticorps anti-anti-idiotypiques sont criblés par une batterie de tests, notamment par un test ELISA anti-idiotypique : les anticorps anti-idiotypiques tels qu'obtenus précédemment sont tapissés dans du tampon carbonate 50 mM pH 9 à une dilution de 10 µg/ml. La surface des puits est ensuite incubée avec de la sérumalbumine à 0,5%. Les surnageants d'hybridomes sont ensuite incubés dans les boîtes et leur présence est révélée par les anticorps anti-immunoglobulines de souris conjugués à la peroxydase.

On vérifie également que les anticorps anti-anti-idiotypiques se lient aux cellules endothéliales angiogéniques ou non angiogéniques, selon les mêmes tests que ceux décrits dans le paragraphe 3.

### 6. Immunohistochimie de coupes de glioblastome

Le glioblastome est une tumeur maligne du cerveau dont la caractéristique majeure est d'être extrêmement vascularisée. Des coupes sériées de pièces opératoires prélevées au cours de la chirurgie de glioblastomes ont été incubées avec des anticorps reconnaissant les cellules musculaires lisses des vaisseaux (SM actin) (Figure 10A) ou les cellules endothéliales (anti-CD 31) (Figure 10B) ou l'anticorps C défini ci -dessus (Figure 10C).

Des coupes d'épaisseur 3 µm de glioblastomes sont déparaffinées.

Ensuite, les sites antigéniques sont démasqués de la façon suivante : on effectue 2 passages de 5 minutes desdites coupes au micro-ondes (puissance maximale) dans Tp Citrate 10 mM pH 6,0 (tri-sodium citrate C₆H₅Na₃O₇, 2H₂O PM 294,10), et on laisse refroidir les lames à température ambiante 30 minutes puis on les place en attente dans du PBS 1X.

Le tampon citrate 10 mM est obtenu comme suit : on mélange 5,9 g de tri-sodium citrate et de l'eau (qsp 2 1) puis le pH est ajusté à 6 avec de l'acide citrique en poudre.

Les sites non spécifiques sont bloqués par mise en présence des coupes susmentionnées avec un sérum bloquant prédilué (sérum de cheval à 2,5%) pendant 20 minutes. On élimine ensuite l'excès de sérum par rinçage dans du PBS pendant 5 minutes.

On incube les coupes pendant 30 minutes avec différents anticorps (dilués dans du PBS contenant 0,1% de BSA), à température ambiante, en chambre humide, et on rince avec du PBS pendant 5 minutes.

On effectue une incubation supplémentaire avec l'anticorps secondaire biotinylé universel prédilué pendant 30 minutes (KIT VECTASTAIN Elite UNIVERSAL RTU).

L'ensemble ainsi obtenu est incubé avec le complexe Elite ABC avidine-biotine-peroxydase pendant 30 minutes, puis on effectue un rinçage au PBS pendant 5 minutes.

On incube avec le réactif AEC prêt à l'emploi (Dako K3464) pendant 10 minutes, et on rince avec de l'eau distillée.

On effectue ensuite un contre-coloriage à l'hématoxyline de Mayer pendant 2 minutes et on rince à l'eau ammoniaquée (2,5/1000) puis à l'eau distillée

On incube ensuite la lame avec du liquide de montage aqueux (Dako faramount S3025) et l'on dépose une lamelle couvre-objet.

### RESULTATS

### I. Etablissement des phénotypes des cellules F/O et F/V.

### Morphologie

Le phénotype des souches F/0 et F/V est maintenu par l'adjonction de VEGF dans le milieu de culture à long terme. Quand on ôte le VEGF (cas de l'absence de VEGF), la morphologie de chacune des souches est différente : monocouche régulière pavimenteuse à confluence pour F/V, les F/0 sont des cellules plus grandes et arrondies.

Quatre heures après l'adjonction du VEGF, seules les cellules F/V subissent un changement de morphologie : les cellules s'allongent, l'espace intercellulaire devenant important (voir Figures 1C et 1D). En revanche, les cellules F/0 ne changent pas de forme (Figures 1A et 1B).

### Expression de gènes

L'expression de VEGFR-2 et du collagène I a été étudiée dans les cellules F/0 et F/V par Northem Blot. Il apparaît que l'expression de VEGFR-2 est augmentée de 4 fois dans les cellules F/V par rapport à F/0. En revanche l'expression de VEGFR-2 est identique dans BREC/0 et BREC/V (Hutchings et al., 2002).

De plus l'expression d'autres gènes a été comparée dans les couples 0 et V des cellules endothéliales de rétine de boeuf (BREC) et de cellules endothéliales de foetus de boeuf (F) (voir Figure 2C)

Par exemple, la fibronectine apparaît plus exprimée par les cellules F/0 que par les cellules F/V (rapport 3,5) alors que son expression est similaire dans les cellules BREC/0 et BRECN.

Par exemple les séquences AK024691, AK 025662, et X85799 sont respectivement 4, 12 et 2,5 fois plus exprimées dans les cellules FV que les cellules F/0

### Angiogenèse in vitro en réponse au VEGF

Les cellules F/0 ou F/V ensemencées sur un gel de collagène-matrigel sont incubées avec 50 ng/ml de VEGF et leur morphologie est examinée 24 heures après. Les cellules F/V stimulées par le VEGF s'organisent en faisceaux comportant une rangée de 3-4 cellules puis en structures tubulaires comme le montre la Figure 3B. En revanche les cellules F/0 restent disposées en amas, qu'elles soient ou non stimulées par le VEGF (Figure 3A).

### Réponse aux traitements anti-angiogéniques

Les cellules FN sont sensibles à l'action mitogène du VEGF (ED 50 = 0,3 ng/ml) alors que les cellules F/0 ne prolifèrent pas sous l'action du VEGF.

Les cellules F/0 et F/V sont ensemencées à basse densité (5000) comme indiqué et trois drogues sont inoculées séparément dont 2 de mécanisme inconnu (Méthoxyestradiol-ME-2 et fumagilline - FUMA) et 1 interférant avec le VEGF-R2 (SU 5416 inhibant l'activité kinase. Il apparaît que les cellules F/0 et FN sont également sensibles à la fumagilline et au méthoxyestradiol (Figure 4) alors que les cellules FN sont plus sensibles au SU 5416. Il apparaît que l'IC50 de ces 2 composés est multipliée par 20 et 50 respectivement pour les cellules F/0.

Ainsi donc l'utilisation de ces cellules permet de pratiquer un criblage de médicaments anti-angiogéniques qui n'affectent pas les cellules endothéliales des vaisseaux sains, non angiogéniques.

### II. Production d'anticorps monoclonaux spécifiques du phénotype angiogénique

### Reconnaissance spécifique des cellules F/V

4 fusions ont été effectuées et 426 hybridomes ont été obtenus. Après un criblage par ELISA, 15 clones ont été sous clonés et 1026 clones analysés dont 6 ont été sélectionnés par ELISA, correspondant à des anticorps monoclonaux se fixant sur F/0 ou F/V. La vérification de la spécificité de ces anticorps monoclonaux a été effectuée par cytométrie de flux.

Il apparaît que certains anticorps se fixent sélectivement aux cellules F/V ou aux cellules F/0 (voir tableau ci-dessous et figure 8 représentant l'analyse de la spécificité des anticorps par cytométrie de flux).

Le tableau ci-dessous indique les pourcentages de cellules reconnues par les différents types d'anticorps.

| type d'anticorps | F/0 | F/V | BREC/0 | BRECN | musculaire |
|---|---|---|---|---|---|
| A | 58 | 10 | 44,7 | 95,9 | 5,4 |
| B | 25 | 36 | 4,2 | 8,4 | 0 |
| C | 3 | 48 | 43 | 63,7 | 3,8 |

3 types d'anticorps peuvent être distingués :
Marquage F/0 > F/V : type A
Marquage F/V > F/0 : type B
Marquage F/O absent : type C

L'anticorps A marque préférentiellement les cellules F/0 (Figure 8 F) et RPE 0 (Figure 9 F).

L'anticorps B marque préférentiellement les cellules F/V (Figures 8 C), et ils ne reconnaissent pas les RPE 0 et RPE V.

L'anticorps C marque exclusivement les cellules F/V (Figure 8 D) et pas les cellules F 0 (Figure 8 H) et ne reconnait pas les RPE 0 et RPE V.

### Prolifération

L'étude de la fonction mitogène des anticorps permet de les classer en 2 catégories : les anticorps de type 3 inhibent la prolifération des cellules FN sans affecter celle des cellules F/0 (Figures 5A et 5B) et les anticorps de type 5 stimulent la prolifération des cellules F/0 sans affecter celle des cellules F/V (Figure 6).

### Tumorigenèse

La figure 7 montre que l'injection des anticorps de type B réduit le volume de la tumeur à chaque temps d'observation.. Le tableau suivant montre les moyennes et les écart-types des volumes des tumeurs observées à 20 jours. On en déduit que l'anticorps de type A n'a pas d'effet sur la tumorigenèse, ce qui est en accord avec les résultats de cytométrie de flux démontrant que cet anticorps ne se fixe pas aux cellules angiogéniques.

En revanche les anticorps de type B et C reconnaissant les cellules de phénotype angiogénique par cytométrie de flux inhibent la croissance tumorale.

| Injection | moyenne (mm3) | écart-type (mm3) |
|---|---|---|
| PBS | 3226 | 1255 |
| Ac A | 2159 | 350 |
| Ac B | 1299 | 368 |
| Ac C | 1866 | 287 |

### III. Immunohistochimie de coupes de glioblastome

Les figures 10A, 10B et 10C montrent
- un marquage avec l'anticorps anti-actine ("smooth muscle cell actin") :marquage de toutes les cellules périvasculaires musculaires lisses;
- un marquage avec l'anticorps anti-CD 31 : marquage de toutes les cellules endothéliales ; et
- un marquage avec l'anticorps C : marquage de quelques cellules endothéliales tumorales.

Par ailleurs, il n'a jamais été détecté de marquage avec l'anticorps C dans des coupes de tissu sain (cerveau, poumon, coeur, rein, foie).

En conclusion l'anticorps susmentionné est donc spécifique des cellules du phénotype angiogénique et ne reconnaît pas les cellules de phénotype non angiogénique.

### RÉFÉRENCES

Battegay, E.J., Rupp, J., Iruela-Arispe, L., Sage, E.H., Pech, M. (1994) PDGF-BB modulates endothelial proliferation and angiogenesis in vitro via PDGF b-receptors. J Cell. Biol. 125, 917-928.
Boudreau, N., Andrews, C., Srebrow, A., Ravanpay, A., Cheresh, D.A. (1997) Induction of the angiogenis phenotype by Hox D3. J Cell. Biol. 139, 257-264.
Brooks, P.C., Lin, J., French, D.L., Quigley, J.P. (1993) Subtractive immunization yields monoclonal antibodies that specifically inhibit metastasis. J Cell. Biol. 122, 1351-1359.
Brooks PC., Clark RA., Cheresh DA. (1994) Requirement of vascular integrin alpha v beta 3 for angiogenesis. Science 264, 569-71.
Concina P, Sordello S., Elhage N., Fournial P., Plouët J., Bayard F., Arnal J-F. (2000) The mitogenic effect of 17 β-Estradiol on in vitro endothelial cell proliferation and on in vivo reendothelialization are both dependent on VEGF. J. Vasc. Res. 37, 202-208.
Gavrieli Y., Sherman Y., Ben-Sasson SA. (1992) Identification of programmed cell death in situ via specific labeling of nuclear DNA fragmentation. J. Cell. Biol. 119, 493-501.
Hutchings, H., Maitre-Boube, M., Tomban-Tink, J., Plouët, J. (2002) Pigment epithelium-derived factor exerts opposite effects on endothelial cells of different phenotypes. BBRC 294, 764-769.
Jonca F., Ortéga N., Gleizes P.-E., Bertrand N., Plouët J. (1997) Cell release of bioactive fibroblast growth factor by exon 6 encoded sequence of vascular endothelial growth factor. J. Biol. Chem. 272, 24203-24209.
Keanney JF., Radbruch A., Liesegang B., Rajewsky K. (1979) A new mouse myeloma cell line that has lost immunoglobulin expression but permits the construction of antibody-secreting hybrid cell lines. J. Immunol. 123, 1548-50.
Kohler et Milstein (1975) Continuous cultures of fused cells secreting antibody of predefined specificity. Nature, Aug 7; 256(5517): 495-7.
Plouët J., Moro F., Bertagnolli S., Coldeboeuf N., Mazarguil H., Clamens S., Bayard F. (1997) Extracellular cleavage of the vascular endothelial growth factor 189-amino acid form by urokinase is required for its mitogenic effect. J Biol. Chem. 272, 13390-96.
Plouët et al. (2001) Angiogenesis, 3, 215-217
Sambrook K., Fritsch E, Maniatis T. (1989) « Molecular cloning : a laboratory manual » Cold Spring Harbor ed. (C. S. H. Laboratory, Ed.) New York.

## Revendications

1. Cellules endothéliales de phénotype angiogénique, présentant les propriétés suivantes :
- elles forment des tubes lorsqu'elles sont mises en présence du facteur de croissance VEGF dans un gel de collagène,
- elles prolifèrent sous l'action du VEGF,
- elles sont protégées de l'apoptose par le VEGF, et
- leur expression de VEGF-R2 est 4 fois supérieure à celle des cellules correspondantes de phénotype non angiogénique,
et **caractérisées en ce qu'**elles sont susceptibles de générer des anticorps présentant une activité anti-tumorale.

2. Cellules endothéliales selon la revendication 1, **caractérisées en ce que** ce sont des cellules endothéliales de vaisseau, notamment des cellules endothéliales d'aorte, de cortex surrénal, de peau, de cerveau, de veine ou d'artère de cordon ombilical.

3. Procédé de préparation de cellules endothéliales de phénotype angiogénique selon la revendication 1, **caractérisé en ce qu'**il comprend les étapes suivantes :
- l'incubation, de cellules endothéliales, notamment prélevées d'une aorte dans un milieu contenant de l'oestradiol et du VEGF, afin d'obtenir des clones de cellules endothéliales de phénotype angiogénique,
- le prélèvement d'un clone à l'aide d'une micropipette, parmi les clones susmentionnés, de cellules endothéliales de phénotype angiogénique, et la culture de ce clone dans un milieu contenant de l'oestradiol et du VEGF jusqu'à l'obtention de la confluence des cellules,
- la sélection des cellules endothéliales de phénotype angiogénique, par vérification du phénotype des cellules obtenues à l'étape précédente, à l'aide de tests de prolifération, de migration ou d'angiogenèse *in vitro.*

4. Procédé de préparation d'un anticorps monoclonal dirigé contre les cellules endothéliales de phénotype angiogénique selon la revendication 1, et inhibant l'angiogenèse, **caractérisé en ce qu'**il comprend les étapes suivantes :
- l'immunisation d'un animal par injection de cellules de phénotype angiogénique selon la revendication 1,
- la fusion entre des myélomes d'un animal et des splénocytes d'un animal afin d'obtenir des hybridomes,
- la mise en culture des hybridomes ainsi obtenus,
- le clonage d'hybridomes, choisis parmi ceux obtenus à l'étape précédente et sécrétant des anticorps contre les cellules de phénotype angiogénique selon la revendication 1,
- la vérification des propriétés d'inhibition de l'angiogenèse des anticorps susmentionnés vis-à-vis des cellules angiogéniques, notamment à l'aide du test de prolifération et/ou de migration et/ou d'angiogenèse *in vitro.*

5. Anticorps monoclonal dirigé contre les cellules endothéliales de phénotype angiogénique selon la revendication 1, et inhibant l'angiogenèse, susceptible d'être obtenu selon le procédé de la revendication 4.

6. Anticorps monoclonal selon la revendication 5, présentant les caractéristiques suivantes :
- il se lie à la surface des cellules endothéliales de phénotype angiogénique, et
- il reconnaît un motif présent exclusivement sur les cellules endothéliales de phénotype angiogénique, notamment un récepteur membranaire.

7. Anticorps anti-idiotypiques dirigés contre des anticorps monoclonaux selon l'une quelconque des revendications 5 ou 6, **caractérisés en ce qu'**ils sont capables d'inhiber les fonctions exercées (inhibition de l'angiogenèse) par lesdits anticorps.

8. Fragments Fab des anticorps monoclonaux selon l'une quelconque des revendications 5 à 6, lesdits fragments étant capables d'inhiber l'angiogenèse.

9. Procédé de préparation des anticorps anti-idiotypiques selon la revendication 7, ledit procédé étant **caractérisé en ce qu'**il comprend les étapes suivantes :
- l'immunisation d'un animal par injection d'anticorps monoclonaux selon la l'une quelconque des revendications 4 5 à 6,
- la fusion entre des myélomes d'un animal et des splénocytes d'un animal, afin d'obtenir des hybridomes,
- la mise en culture des hybridomes ainsi obtenus,
- le clonage d'hybridomes, choisis parmi ceux obtenus à l'étape précédente et sécrétant des anticorps dirigés contre les anticorps monoclonaux susmentionnés, utilisés pour l'immunisation, lesdits anticorps monoclonaux étant dirigés contre les cellules de phénotype angiogénique, et
- la vérification des propriétés d'inhibition des anticorps susmentionnés vis-à-vis de la fonction d'inhibition de l'angiogenèse des anticorps selon l'une quelconque des revendications 4 5 à 6, notamment à l'aide du test de prolifération et/ou de migration et/ou d'angiogenèse *in vitro.*

10. Procédé de préparation des anticorps anti-anti-idiotypiques capables d'inhiber l'angiogenèse dirigés contre des cellules endothéliales de phénotype angiogénique selon la revendication 1, **caractérisé en ce qu**'il comprend les étapes suivantes :
- l'immunisation d'un animal par injection d'anticorps anti-idiotypiques selon la revendication 7,
- la fusion entre des myélomes d'un animal et des splénocytes d'un animal afin d'obtenir des hybridomes,
- la mise en culture des hybridomes ainsi obtenus,
- le clonage d'hybridomes, choisis parmi ceux obtenus à l'étape précédente et sécrétant des anticorps anti-anti-idiotypiques dirigés contre les cellules de phénotype angiogénique, et
- la vérification des propriétés d'inhibition de l'angiogenèse des anticorps anti-anti-idiotypiques susmentionnés, notamment à l'aide du test de prolifération et/ou de migration et/ou d'angiogenèse in vitro.

11. Anticorps anti-anti-idiotypiques dirigés contre des cellules endothéliales de phénotype angiogénique selon la revendication 1, susceptibles d'être obtenu selon le procédé de la revendication 10, **caractérisés en ce qu'**ils sont capables d'inhiber l'angiogenèse.

12. Composition pharmaceutique, **caractérisée en ce qu'**elle contient, à titre de substance active, un inhibiteur de l'angiogenèse, choisi parmi un anticorps selon l'une des revendications 5 ou 6, un fragment Fab selon la revendication 8, en association avec un vecteur pharmaceutiquement acceptable, ladite composition pharmaceutique étant susceptible d'être administrée à raison d'environ 0,01 à environ 20 mg/kg/injection.

13. Composition vaccinale, **caractérisée en ce qu'**elle comprend, à titre de substance active un anticorps monoclonal selon l'une des revendications 5 ou 6, ou des fragments Fab selon la revendication 8, ou un anticorps anti-anti-idiotypique selon la revendication 11, en association avec un adjuvant pharmaceutiquement acceptable.

14. Utilisation d'un inhibiteur de l'angiogenèse, choisi parmi un anticorps selon l'une des revendications 5 ou 6, un fragment Fab selon la revendication 8, ou un anticorps anti-anti-idiotypique selon la revendication 11, pour la préparation d'un médicament destiné au traitement de pathologies nécessitant l'inhibition de la prolifération endothéliale, notamment dans le cadre des pathologies suivantes : les cancers, la dégénérescence musculaire liée à l'âge, les rétinopathies diabétiques, la polyarthrite rhumatoïde, les angiomes, les angiosarcomes, en particulier la maladie de Castelman et le sarcome de Kaposi.

15. Utilisation d'un inhibiteur de l'angiogenèse, choisi parmi un anticorps selon l'une des revendications 5 ou 6, un fragment Fab selon la revendication 8, ou un anticorps anti-anti-idiotypique selon la revendication 11, pour la préparation d'un médicament destiné au traitement de pathologies nécessitant l'inhibition de l'activation endothéliale, notamment dans le cadre des pathologies suivantes : le rejet d'allogreffe et de xénogreffe, les acrocyanoses, les sclérodermies, ou dans le cadre de la préparation de greffons entre le prélèvement et la transplantation.

## Claims

1. Endothelial cells with an angiogenic phenotype, having the following properties:
- they form tubes in presence of growth factor VEGF in a collagen gel,
- they proliferate under the action of VEGF,
- they are protected from apoptosis by VEGF, and
- the expression of VEGFR-2 is increased 4-fold in comparison with cells with a non-angiogenic phenotype
**characterized in that** they are liable to generate antibodies having anti-tumoral properties.

2. Endothelial cells according to claim 1, **characterized in that** they are endothelial cells of vessels, in particular endothelial cells of the aorta, adrenal cortex, skin, cerebrum, retina, veins or umbilical cord artery.

3. A process for preparing endothelial cells with an angiogenic phenotype according to claim 1, **characterized in that** it comprises the following stages:
- incubation of endothelial cells, in particular removed from an aorta in a medium containing oestradiol and VEGF, in order to obtain clones of endothelial cells with an angiogenic phenotype,
- removal of a clone using a micropipette, from the abovementioned clones of endothelial cells with an angiogenic phenotype, and the culture of this clone in a medium containing oestradiol and VEGF until cell confluence is obtained,
- selection of the endothelial cells with an angiogenic phenotype, by verification of the phenotype of the cells obtained in the preceding stage, using proliferation, migration or in vitro angiogenesis tests.

4. A process for preparing a monoclonal antibody directed against endothelial cells with an angiogenic phenotype according to claim 1, and inhibiting angiogenesis, **characterized in that** it comprises the following stages:
- immunization of an animal by injection of cells with an angiogenic phenotype according to claim 1,
- fusion between myelomas of an animal and splenocytes of an animal in order to obtain hybridomas,
- culture of the hybridomas thus obtained,
- cloning of hybridomas, chosen from those obtained in the preceding stage and secreting antibodies against cells with an angiogenic phenotype according to claim 1,
- verification of the angiogenesis-inhibiting properties of the abovementioned antibodies vis-à-vis angiogenic cells, in particular using the proliferation and/or migration and/or *in vitro* angiogenesis test.

5. Monoclonal antibody directed against endothelial cells with an angiogenic phenotype according to claim 1, and inhibiting angiogenesis, liable to be obtained by the process of claim 4.

6. Monoclonal antibody according to claim 5, having the following characteristics:
- it binds to the surface of endothelial cells with an angiogenic phenotype, and
- it recognizes a unit present exclusively on endothelial cells with an angiogenic phenotype, in particular a membrane receptor.

7. Anti-idiotypic antibodies directed against monoclonal antibodies according to claim 5 or 6, **characterized in that** they are capable of inhibiting the functions performed (inhibition of angiogenesis) by said antibodies.

8. Fab fragments of the monoclonal antibodies according to one of claims 5 to 6, said fragments being capable of inhibiting angiogenesis

9. A process for preparing anti-idiotypic monoclonal antibodies according to claim 7, said process being **characterized in that** it comprises the following stages:
- immunization of an animal by injection of monoclonal antibodies according any of claims 5 or 6 ,
- fusion between myelomas of an animal and splenocytes of an animal in order to obtain hybridomas,
- culture of the hybridomas thus obtained,
- cloning of hybridomas, chosen from those obtained in the preceding stage and secreting antibodies directed against the abovementioned monoclonal antibodies, used for the immunization, said monoclonal antibodies being directed against cells with an angiogenic phenotype, and
- verification of the inhibition properties of the abovementioned antibodies vis-à-vis the function of inhibition of the angiogenesis of the antibodies according to anyone of claims 5 or 6, in particular using the proliferation and/or migration and/or *in vitro* angiogenesis test.

10. A process for preparing an anti-anti idiotypic monoclonal antibody capable of inhibiting angiogenesis, directed against endothelial cells with an angiogenic phenotype according to claim 1, **characterized in that** it comprises the following stages:
- immunization of an animal by injection of anti-diotypic antibodies according to claim 7,
- fusion between myelomas of an animal and splenocytes of an animal in order to obtain hybridomas,
- culture of the hybridomas thus obtained,
- cloning of hybridomas, chosen from those obtained in the preceding stage and secreting anti-anti-idiotypic antibodies directed against cells with an angiogenic phenotype, and
- verification of the properties of inhibition of the angiogenesis of the abovementioned anti-anti-idiotypic antibodies, in particular using the proliferation and/or migration and/or in vitro angiogenesis test.

11. Anti-anti-idiotypic antibodies directed against endothelial cells with an angiogenic phenotype according to claim 1, liable to be obtained by the process of claim 10, **characterized in that** they are capable of inhibiting angiogenesis.

12. Pharmaceutical composition, **characterized in that** it contains, as active ingredient, an angiogenesis inhibitor, chosen from an antibody according to one of claims 5 or 6, a Fab fragment according to claim 8, in combination with a pharmaceutically acceptable vector, said pharmaceutical composition being capable of being administered at a rate of approximately 0.01 to approximately 20 mg/kg/injection.

13. A vaccine composition, **characterized in that** it comprises, as active ingredient a monoclonal antibody according to one of claims 5 or 6, or Fab fragments according to claim 8, or an anti-anti-idiotypic antibody according to claim 11, in combination with a pharmaceutically acceptable adjuvant.

14. Use of an angiogenesis inhibitor, chosen from an antibody according to one of claims 5 or 6, a Fab fragment according to claim 8, or an anti-anti-idiotypic antibody according to claim 11, for preparing a medicament intended for the treatment of pathologies requiring inhibition of endothelial proliferation, in particular within the framework of the following pathologies: cancers, muscle degeneration linked with age, diabetic retinopathies, rheumatoid polyarthritis, angiomas, angiosarcomas, in particular Castelman's disease and Kaposi's sarcoma.

15. Use of an angiogenesis inhibitor, chosen from an antibody according to one of claims 5 or 6, a Fab fragment according to claim 8, or an anti-anti-idiotypic antibody according to claim 11, for preparing a medicament intended for the treatment of pathologies requiring the inhibition of endothelial activation, in particular within the framework of the following pathologies: allograft and xenograft rejection, acrocyanosis, sclerodermas, or within the framework of the preparation of grafts between removal and the transplantation.

## Patentansprüche

1. Endotheliale angiogen-phänotypische Zellen, mit den folgenden Eigenschaften :
- Sie bilden Rohren, wenn sie mit dem VEGF Wachstumsfaktor in einem Kollagen-Gel konfrontiert sind,
- Sie vermehren sich durch den VEGF,
- Sie sind gegen Apoptose durch den VEGF geschützt, und
- Ihre Expression von VEGF ist 4 mal über die der entsprechenden Zellen mit nichtangiogenischem Phänotyp,
und die **dadurch gekennzeichnet sind, daß** sie in der Lage sind, Antikörper mit einer antitumoralen Aktivität zu generieren.

2. Endotheliale Zellen nach Anspruch 1, daduch **gekennzeichnet**, daß sie endotheliale Zellen von Gefäßen, und zwar besonders endotheliale Zellen von Aorta, adrenalem Kortex, Haut, Hirn, Vene oder Ader vom Nabelschnur sind.

3. Verfahren für die Herstellung von endothelialen angiogen-phänotypischen Zellen nach Anspruch 1, **dadurch gekennzeichnet, daß** es die folgende Schritte beinhaltet :
- die Inkubation von endothelialen Zellen, insbesondere die aus einer Aorta in einem Medium, das Oestradiol und VEGF enthaltet, entnommen sind, um Klone von angiogen-phenotypischen endothelialen Zellen zu erhalten,
- die Entnahme eines Klones mit einer Mikropipette, und zwar unter den obengenannten Klonen, von angiogen-phänotypischen endothelialen Zellen, und die Kultur dieses Klones in einem Medium, das Oestradiol und VEGF enthaltet, bis man eine Konfluenz der Zellen erreicht,
- die Selektion von angiogen-phänotypischen endothelialen Zellen durch Überprüfung des Phänotyps von Zellen, die in den vorhergehenden Schritt bekommt wurden, und zwar mit *in vitro* Proliferations-, Migrations- oder Angiogenese-Tests.

4. Verfahren für die Herstellung eines monoklonales Antikörpers gegen die angiogen-phänotypische endotheliale Zellen nach Anspruch 1, und die die Angiogenese hemmen, **dadurch gekennzeichnet, daß** es die folgenden Schritte beinhaltet :
- die Immunisierung eines Tieres durch Injektion von angiogen-phänotypischen Zellen nach Anspruch 1,
- die Fusion zwischen Myelomen eines Tieres und Splenozyten eines Tieres, um Hybridome zu erhalten,
- die Kultivierung der in dieser Weise erhaltenen Hybridome,
- die Klonierung von Hybridomen, die unter denen, die in dem vorhergehenden Schritt erhalten wurden, gewählt werden, und die auch Antikörper gegen angiogen-phenotypische Zellen nach Anspruch 1 sezernieren,
- die Überprüfung der Angiogenese-Hemmungseigenschaften der obengenannten Antikörper gegenüber den angiogenischen Zellen, insbesondere mit *dem in vitro* Proliferations- und/oder Migrations- und/oder Angiogenese-Test.

5. Monoklonaler Antikörper gegen die angiogen-phänotypische endotheliale Zellen nach Anspruch 1, die auch die Angiogenese hemmen und die nach dem Verfahren der Anspruch 4 erhalten werden können.

6. Monoklonaler Antikörper nach Anspruch 5, der die folgenden Eigenschaften aufweisen :
- Er bindet sich zur Ebene der angiogen-phänotypischen endothelialen Zellen, und
- Er erkennt eine Einheit, die ausschließlich auf die angiogen-phänotypischen endothelialen Zellen anwesend ist, insbesondere einen Membran-Rezeptor.

7. Anti-idiotypische Antikörper, die gegen monoklonale Antikörper nach irgendwelchem Anspruch 5-6 orientiert sind, **dadurch gekennzeichnet, daß** sie in der Lage sind, die Funktion dieser Antikörper (Angiogenese-Hemmung) zu hemmen.

8. Fab Fragmente der monoklonalen Antikörper nach irgendwelcher Anspruch 5-6, worin diese Fragmente fähig sind, die Angiogenese zu hemmen.

9. Verfahren für die Herstellung von anti-idiotypischen Antikörper nach Anspruch 7, **dadurch gekennzeichnet, daß** es die folgenden Schritte einschließt:
- Die Immunisierung eines Tieres durch Injektion von monoklonalen Antikörpern nach irgendwelcher Anspruch 5-6,
- Die Fusion zwischen den Myelomen eines Tieres und den Splenozyten eines Tieres, um Hybridome zu erhalten,
- Die Kultivierung der so erhaltenen Hybridomen,
- Die Klonierung von Hybridomen, die unter denen, die im vorhergehenden Schritt erhalten wurden, gewählt werden, und die auch Antikörper gegen die obengenannten monoklonalen Antikörper, die für die Immunisation verwendet werden, sezernieren, worin die obengenannten monoklonalen Antikörper gegen die angiogen-phänotypischen Zellen orientiert sind, und
- Die Überprüfung der Hemmungseigenschaften der obengenannten Antikörper gegen die Angiogenese-Hemmungsfunktion der Antikörper nach irgendwelcher Anspruch 4-6, besonders mit dem *in vitro* Proliferations- und/oder Migrations- und/oder Angiogenese-Test.

10. Verfahren für die Herstellung von anti-anti-idiotypischen Antikörper, die fähig sind, die Angiogenese zu hemmen, und die gegen angiogen-phänotypische endotheliale Zellen nach Anspruch 1 orientiert sind, **dadurch gekennzeichnet, daß** es die folgenden Schritte einschließt :
- Die Immunisierung eines Tieres durch Injektion von anti-idiotypischen Antikörpern nach Anspruch 7,
- Die Fusion zwischen Myelomen eines Tieres und Splenozyten eines Tieres um Hybridome zu erhalten,
- Die Kultivierung der so erhaltenen Hybridomen,
- Die Klonierung von Hybridomen, die unter denen, die im vorhergehenden Schritt erhalten wurden, gewählt werden, und die auch anti-anti-idiotypische Antikörper, die gegen die angiogen-phänotypische Zellen orientiert sind, sezernieren, und
- Die Überprüfung der Angiogenese-Hemmungseigenschaften von den obengenannten anti-anti-idiotypischen Antikörpern, insbesondere mit dem *in vitro* Proliferations- und/oder Migrations- und/oder Angiogenese-test.

11. Anti-anti-idiotypische Antikörper gegen angiogen-phänotypische endotheliale Zellen nach Anspruch 1, die mit dem Verfahren nach Anspruch 10 erhalten können, **dadurch gekennzeichnet, daß** sie fähig sind, die Angiogenese zu hemmen.

12. Pharmazeutische Zubereitung, **dadurch gekennzeichnet, daß** sie als Aktivsubstanz einen Angiogenese-Hemmer enthält, der unter einem Antikörper nach irgendwelcher Anspruch 5 oder 6 oder ein Fab Fragment nach Anspruch 8, gemeinsam mit einem pharmazeutisch annehmbaren Vektor gewählt wurde, worin die obengenannte pharmazeutische Zubereitung zwischen ca. 0,01 und ca. 20 mg/kg/Injektion verabreicht werden kann.

13. Impfstoff Zusammensetzung, **dadurch gekennzeichnet, daß** sie als Aktivsubstanz einen monoklonalen Antikörper nach irgendwelcher Anspruch 5 oder 6 oder Fab Fragmente nach Anspruch 8, oder einen anti-anti-idiotypischen Antikörper nach Anspruch 11, gemeinsam mit einem pharmazeutisch annehmbaren Adjuvans enthält.

14. Verwendung eines Angiogenese-Hemmers, der unter einem Antikörper nach irgendwelcher Anspruch 5 oder 6, einem Fab Fragment nach Anspruch 8 oder einem anti-anti-idiotypischen Antikörper nach Anspruch 11 gewählt wurde, für die Herstellung eines Arzneimittels für die Behandlung von Pathologien, die die Hemmung der endothelialen Proliferation bedürfen, und zwar insbesondere in den folgenden Pathologien : Krebse, Alter-verursachte Muskeldegeneration, diabetische Retinopathien, Polyarthritis rheumatica, Angiome, Angiosarkome, insbesondere die Castelmansche Krankheit und das Kaposi-Sarkom.

15. Verwendung eines Angiogenese-Hemmers, der unter einem Antikörper nach irgendwelcher Anspruch 5 oder 6, einem Fab Fragment nach Anspruch 8 oder einem anti-anti-idiotypischen Antikörper nach Anspruch 11 gewählt wurde, für die Herstellung eines Arzneimittels für die Behandlung von Pathologien, die die Hemmung der endothelialen Aktivierung bedürfen, und zwar insbesondere in den folgenden Pathologien : die Abstoßung eines Homo- und Xeno-transplantates, die Akrozyanosen, die Sklerodermien, oder in der Vorbereitung von Transplantaten zwischen der Entnahme und der Transplantation.
